# EUROPEAN PATENT APPLICATION

(11) **EP 4 670 752 A1**
(43) Date of publication of application: **31.12.2025**
(21) Application number: 24184992.6
(22) Date of filing: 27.06.2024
(51) Int. Cl.: A61M 1/00

(54) **NEGATIVE PRESSURE WOUND THERAPY**

(71) Applicant: Mölnlycke Health Care AB, 402 52 Göteborg (SE)
(72) Inventor: UVEBORN, Johan, 436 40 Askim (SE); BENGTSSON, Erik, 431 41 Mölndal (SE); VANNAS, Alexander, 421 51 Göteborg (SE); DOLANGE, Guillaume, 412 78 Mölndal (SE)
(74) Representative: Zacco Sweden AB

(57) **Abstract**

An apparatus and related aspects for providing reduced pressure to a tissue site are disclosed. The apparatus comprises a source of negative pressure configured to provide negative pressure via a first fluid flow path from an inlet of the source of negative pressure to a wound site. The apparatus further comprises a canister for receiving fluids from the wound site, where the canister has a canister outlet adapted to be fluidly coupled to the inlet of the source of negative pressure and a canister inlet adapted to be fluidly coupled to the wound site. The apparatus further comprises an electronically controllable valve configured to release negative pressure from the wound site via a second fluid flow path, a first pressure sensor configured to monitor a pressure level in the first fluid flow path, and control circuitry operatively connected to the source of negative pressure, the electronically controllable valve, and to the pressure sensor. The control circuitry is configured to estimate an amount of exudate to be moved from the wound site via a portion of the first fluid flow path to the canister, and control a duration of a flushing period and/or a duration of an inter-flushing period based on the estimated amount of exudate. The flushing period defines a time period during which the electronically controllable valve is opened and the source of negative pressure is activated for transporting fluid from the wound site to the canister, and the inter-flushing period defines a time between two consecutive flushing periods.

## Description

### TECHNICAL FIELD

The herein disclosed embodiments generally relate to negative pressure wound therapy (NPWT). In particular, the herein disclosed embodiments relate to apparatuses for providing negative pressure to a wound site, methods for controlling an apparatus for providing negative pressure to a wound site, and thereto related computer program products, computer-readable storage media, and wound treatment systems.

### BACKGROUND

Negative pressure wound therapy (NPWT) is a technique that promotes healing of e.g. surgical, acute and chronic wounds by the application of a sub-atmospheric pressure ("negative pressure") to the wound site, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

### SUMMARY

The herein disclosed technology seeks to mitigate, alleviate or eliminate one or more of the deficiencies and disadvantages in the prior art to address various problems relating to dynamic control of the pressure regulations cycles of NPWT systems.

In particular, it is an object of some embodiments disclosed herein to provide an NPWT apparatus that is capable of adapting the timing and/or duration of the flushing/venting sequences automatically so to provide a more versatile solution that is applicable in a wider range of scenarios.

Various aspects and embodiments of the disclosed technology are defined below and in the accompanying independent and dependent claims.

An aspect of the disclosed technology comprises an apparatus for providing negative pressure to a wound site. The apparatus comprises a source of negative pressure configured to provide negative pressure via a first fluid flow path from an inlet of the source of negative pressure to a wound site. The apparatus further comprises a canister for receiving fluids from the wound site, where the canister has a canister outlet adapted to be fluidly coupled to the inlet of the source of negative pressure and a canister inlet adapted to be fluidly coupled to the wound site. The apparatus further comprises an electronically controllable valve configured to release negative pressure from the wound site via a second fluid flow path, a first pressure sensor configured to monitor a pressure level in the first fluid flow path, and control circuitry operatively connected to the source of negative pressure, the electronically controllable valve, and to the first pressure sensor. The control circuitry is configured to estimate an amount of exudate to be moved from the wound site via a portion of the first fluid flow path to the canister, and control a duration of a flushing period and/or a duration of an inter-flushing period based on the estimated amount of exudate. The flushing period defines a time period during which the electronically controllable valve is opened and the source of negative pressure is activated for transporting fluid from the wound site to the canister, and the inter-flushing period defines a time between two consecutive flushing periods.

Another aspect of the disclosed technology comprises a (computer implemented) method for operating an apparatus for providing negative pressure to a wound site. The apparatus comprises a source of negative pressure configured to provide negative pressure via a first fluid flow path from an inlet of the source of negative pressure to a wound site, a canister for receiving fluids from the wound site, and an electronically controllable valve configured to release negative pressure from the wound site via a second fluid flow path. The method comprises estimating an amount of exudate to be moved from the wound site via a portion of the first fluid flow path to the canister, and controlling a duration of a flushing period and/or a duration of an inter-flushing period based on the estimated amount of exudate. The flushing period defines a time period during which the electronically controllable valve is opened and the source of negative pressure is activated for transporting fluid from the wound site to the canister, and the inter-flushing period defines a time between two consecutive flushing periods. With this aspect of the disclosed technology, similar advantages and preferred features are present as in the other aspects.

Another aspect of the disclosed technology comprises a computer program product comprising instructions which, when the program is executed by a computing device of an apparatus for providing reduced pressure to a wound site, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing reduced pressure to a wound site, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

Another aspect of the disclosed technology comprises a wound treatment system comprising apparatus according to any one of the embodiments disclosed herein, a wound cover for creating a sealed space defined in part by the wound site, and a tubing assembly defining the first fluid flow path and the second fluid flow path. With this aspect of the disclosed technology, similar advantages and preferred features are present as in the other aspects.

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

An advantage of some embodiments is that the energy consumption of the apparatus may be reduced as the apparatus is capable of adaptively controlling the frequency of flushing sequences during use.

An advantage of some embodiments is that there is no need for complex configurations for the timing of the flushing sequences that have to be manually set beforehand based on an assessment of the wound as the apparatus is capable of doing so automatically during run-time, thereby increasing user-friendliness.

An advantage of some embodiments is that the apparatus is rendered more versatile and capable of automatically adapting to various wound treatment scenarios without different wounds (high-exuding wounds, low-exuding wounds, and non-exuding wounds).

An advantage of some embodiments is that noise generation due to excessive and unnecessary pump activations may be reduced for low-exuding or non-exuding wounds.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2 is a schematic graph of negative pressure over time, where an increased amount of exudate is detected based on a pressure change rate as output by an exudate lumen pressure sensor and an inter-flushing period is shortened, in accordance with some embodiments.
Fig. 3 is a schematic graph of negative pressure over time, where an increased amount of exudate is detected based on a pressure change rate as output by an exudate lumen pressure sensor and a flushing period is prolonged, in accordance with some embodiments.
Fig. 4 is a schematic graph of negative pressure over time, where a decreased amount of exudate is detected based on a pressure change rate as output by an exudate lumen pressure sensor and an inter-flushing period is prolonged, in accordance with some embodiments.
Fig. 5 is a schematic graph of negative pressure over time, where a decreased amount of exudate is detected based on a pressure change rate as output by an exudate lumen pressure sensor and a flushing period is shortened, in accordance with some embodiments.
Fig. 6 is a schematic graph of negative pressure over time, where an increased amount of exudate is detected based a comparison between an output of the exudate lumen pressure sensor with the output from an air lumen sensor, and an inter-flushing period is shortened, in accordance with some embodiments.
Fig. 7 is a schematic graph of negative pressure over time as output by the air lumen sensor in the scenario depicted in Fig. 6, in accordance with some embodiments.
Fig. 8 is a schematic graph of negative pressure over time, where an decreased amount of exudate is detected based a comparison between an output of the exudate lumen pressure sensor with the output from an air lumen sensor, and an inter-flushing period is prolonged, in accordance with some embodiments.
Fig. 9 is a schematic graph of negative pressure over time as output by the air lumen sensor in the scenario depicted in Fig. 8, in accordance with some embodiments.
Fig. 10 is a schematic graph of negative pressure over time, where an increased amount of exudate is detected based a number of activations of the source of negative pressure during an inter-flushing period resulting in a prolonged inter-flushing period, in accordance with some embodiments.
Fig. 11 is a schematic graph of negative pressure over time, where an increased amount of exudate is detected based a number of activations of the source of negative pressure during an inter-flushing period resulting in a shortened flushing period, in accordance with some embodiments.
Fig. 12 is a schematic flowchart representation of a method for operating an apparatus for providing negative pressure to a wound site in accordance with some embodiments.

### DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The first signal and the second signal are both signals, but they are not the same signal.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

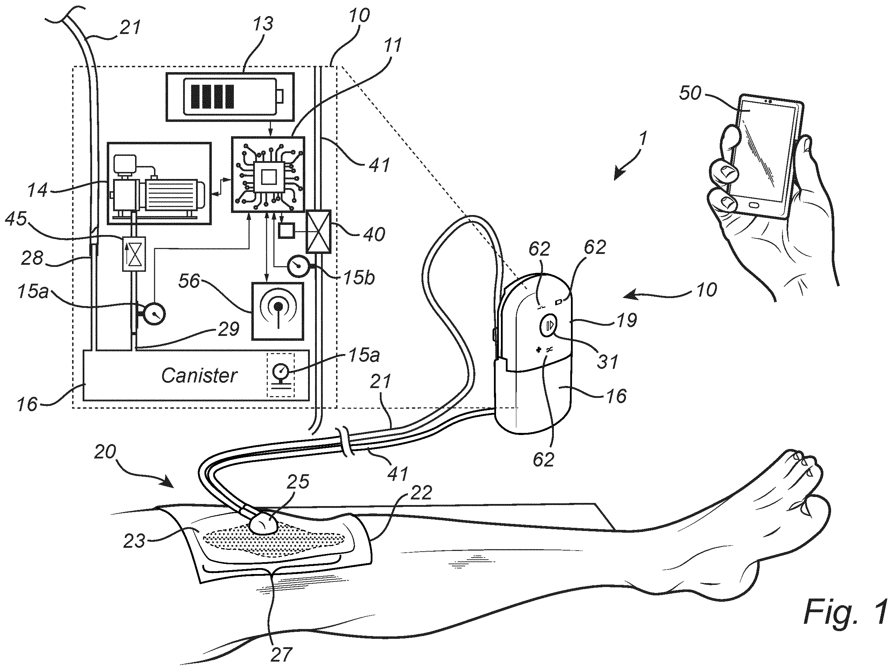

Turning now to the drawings, and to Fig. 1 in particular, there is schematically illustrated a wound treatment system 1 in accordance with some embodiments. The depicted wound treatment system 1 may be referred to as an "NPWT system" 1, "reduced pressure wound treatment system" 1, or "negative pressure wound treatment system" 1. In particular, the depicted wound treatment system 1 may be referred to as a dual lumen wound treatment system 1. The wound treatment system 1 comprises an apparatus 10 for providing negative pressure to a wound site 27 (or otherwise referred to as a tissue site 27). The wound treatment system 1 further comprises a wound cover 22 that is adapted to create a sealed space 23 defined in part by a wound surface, such as at the skin of a user/person, at or around a wound of the user/person.

Further, the apparatus 10 (may also be referred to as an NPWT device 10) is fluidly connected to the wound cover 22 using a tubing assembly with a first tubing 21 at least partly defining a first fluid flow path and a second tubing 41 at least partly defining a second fluid flow path. The tubing 21, 41 may be of any suitable flexible tubing fabricated from elastomeric or polymeric materials. The tubing 21, 41 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing assembly may accordingly comprise two individual tubes 21, 41.

The expression "wound cover" as used herein should be interpreted broadly as any wound site member, e.g. it can be a film sealed around a periphery of a wound site 27, where a wound filler may be used to fill the wound volume prior to the application of such wound cover. Wound cover may also refer to a backing layer of a wound dressing 20 comprising an additional layer(s) such as for example an absorbent layer and/or a spacer layer and/or a tissue interface layer.

The apparatus 10 comprises a source of negative pressure 14 configured to provide negative pressure (i.e. sub-atmospheric pressure) via a first fluid flow path from an inlet of the source of negative pressure 14 to the wound dressing 20, the wound site 27, the sealed space 23, or under the wound cover 22. Thus, the term "to provide negative pressure via a first fluid flow path to a wound site" may be understood as "to provide negative pressure to a wound dressing, "to provide negative pressure to a sealed space defined by a wound cover and a wound site", or "to provide negative pressure under a wound cover". The first fluid flow path ("exudate lumen") is at least partly defined by the first tubing 21. The wound treatment system 1 further comprises a second fluid flow path that fluidly connects the wound site, or the sealed space 23 created by the wound cover 22, to the ambient atmosphere or a fluid reservoir (not shown) via an electronically controllable valve 40. The second fluid flow path ("air lumen") is at least partly defined by the second tubing 41. Accordingly, the fluid flow path from the inlet of the source of negative pressure 14 to the wound site 27 may be construed as a first fluid flow path or "exudate lumen", while the fluid flow path from the ambient atmosphere or fluid reservoir to the wound site 27 via the electronically controllable valve 40 may be construed as a second fluid flow path or "air lumen".

The source of negative pressure 14 is indicated as a pump in the figure. In some embodiments, the source of negative pressure 14 comprises a negative pressure pump that together with a motor is adapted for establishing a negative pressure when the source of negative pressure 14 is operating, i.e. when it is turned on, in an active state, or simply "active". The source of negative pressure 14 may comprise any type of pump and motor that are biocompatible or otherwise suitable for use in an NPWT setting and capable of maintaining or drawing adequate and therapeutic vacuum levels. Preferably, the negative pressure level to be achieved is a value within a range between about -20 mmHg and about -300 mmHg. In some embodiments, a negative pressure range between about -80 mmHg and about -140 mmHg is used. In some embodiments, a negative pressure range between about -115 mmHg and about -135 mmHg is used. In some embodiments, the negative pressure pump is a diaphragm pump, a peristaltic pump, or the like, in which a motor or actuator (e.g., piezoelectric actuator) causes the moving parts to draw fluid from the wound site 27.

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22 or dressing 20. In many cases, the local ambient pressure may also be the atmospheric pressure at which a patient is located. Thus, the "negative pressure" may be understood as a pressure difference between the ambient pressure and the pressure under the wound cover, where ambient pressure is generally set as 0 mmHg. Unless otherwise indicated, values of pressure stated herein are gauge pressures. Similarly, references to increases in negative pressure typically refer to a decrease in absolute/gauge pressure, while decreases in negative pressure typically refer to an increase in absolute/gauge pressure.

Further, in some embodiments the apparatus 10 comprises a canister 16 fluidly connected to the negative pressure source 14. In other words, the negative pressure source 14 is fluidly coupled to the canister 16 and configured to draw a negative pressure within the canister 16. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between two components such as between the canister 16 and the negative pressure source 14 or the canister 16 and the wound dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14. The canister 16 may form a part of the first fluid flow path. Furthermore, the apparatus 10 may comprise a one-way valve 45 between the canister 16 and the source of negative pressure 14 to prevent involuntary backflow of air from the source of negative pressure 14 to the wound site 27.

In some embodiments, the apparatus comprises a housing 19 enclosing the negative pressure source 14. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

In some embodiments, the apparatus 10 comprises a power source, such as e.g. a battery 13 for powering the apparatus 10. The battery 13 may preferably be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions).

Furthermore, the apparatus 10 comprises a first pressure sensor 15a configured to detect, measure, or sense a pressure level in the first fluid flow path ("exudate lumen"). Moreover, the first pressure sensor 15a arranged in fluid connection with the inlet of the source of negative pressure 14 or the canister 16. In particular, the first pressure sensor 15a may be arranged proximal to the inlet of the source of negative pressure 14 (e.g., in connection with a flow path between the canister 16 and the source of negative pressure 14). In the depicted embodiment of Fig. 1, the apparatus 10 comprises a first pressure sensor 15a arranged within the housing 19 in a fluid flow path between the canister 16 and the source of negative pressure 14 (i.e., in fluid connection with the first fluid flow path). However, as readily understood by a person skilled in the art, the first pressure sensor 15a may be located at any other suitable location to sense or detect a pressure within the fluid flow path between the source of negative pressure 14 and the wound site 27. For example, the first pressure sensor 15a may be arranged within the canister 16 as indicated by the broken line box in Fig. 1.

The apparatus may 10 further comprise a second pressure sensor 15b arranged in fluid connection with the second fluid flow path ("air lumen"). In particular, the apparatus 10 may comprise a second pressure sensor 15b configured to detect, measure, or sense a pressure level in the second fluid flow path. In the depicted embodiment the second pressure sensor 15b is arranged within the housing 19 downstream of the electronically controllable valve 40 (i.e., towards the wound site 27 from the valve 40). However, as readily understood by the skilled person in the art, the second pressure sensors 15b may be arranged at any other suitable location to detect, measure or sense a pressure level within the second fluid flow path ("air lumen"). In the following, the first pressure sensor 15a may be referred to as an "exudate lumen sensor" and the second pressure sensor 15b may be referred to as an "air lumen sensor".

The apparatus 10 further comprises control circuitry 11 (may also be referred to as "a control unit", "a controller", or the like) operatively connected to the battery 13, the source of negative pressure 14, the electronically controllable valve 40, and the pressure sensors 15a, 15b. The control circuitry 11 is configured to control operation of the source of negative pressure 14 and the electronically controllable valve 40. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor(s) 11 whereby the apparatus 10 is operative to execute any one of the method steps or functions disclosed herein. The term "electronically controllable" should in present context be understood as that one can control the unit or component that is "electronically controllable" with electric signals (control signals), and in particular with electric signals that are output from the control circuitry 11.

During use of the apparatus 10, the wound cover 22 is arranged at a wound site 27 of the user/patient, forming the sealed space 23. Tubing 21 is provided to fluidly connect the wound cover 22 to the inlet port 28 of the apparatus 10. The apparatus 10 is then activated, e.g. by a user pressing a start/pause button 31 of a user-interface of the apparatus 10. The button 31 may for example be located on the housing 19 of the apparatus 10. The source of negative pressure 14 is thereby activated. When activated, and the valve 40 is closed, the source of negative pressure 14 will start to evacuate air through the canister 16, the inlet port 28, the tubing 21 and the sealed space 23 formed by the wound cover 22. Accordingly, a negative pressure will be created within the sealed space 23. In some embodiments, the control circuitry 11 is configured to control the source of negative pressure 14 so to establish and maintain a negative pressure level (as measured by the exudate lumen sensor 15a and/or as measured by the air lumen sensor 15b) in a negative pressure range. It should be noted that during pressure regulation or during a pressure regulation period (maintenance of negative pressure at the wound site 27), only the output from one of the sensor's 15a, 15b may be actively used to control the pressure level or the output from both sensors 15a, 15b may be actively used to control the pressure level. In some embodiments, only the second pressure sensor's 15b output is used during pressure regulation. In some embodiments, only the first pressure sensor's 15a output is used during pressure regulation. The negative pressure range may for example be -20 mmHg to -300 mmHg, -80 to -180 mmHg, -100 to -150 mmHg, -110 to -140 mmHg, or -115 to -135 mmHg. The negative pressure range may for example be selected in view of the type of wound and/or the patient.

In case a liquid has been formed at the wound site 27, this liquid from the wound site 27 may at least partly be "drawn" from the wound site, through the tubing 21, the inlet port 28 and into the canister 16. The amount of liquid (herein referred to as "exudate") that is drawn from the wound and collected in the canister 16 will depend on the type of wound that is being treated, the duration of the treatment, as well as the type of wound dressing 20 used. For example, in case an absorbent dressing is used, the exudate may be absorbed and collected both in the canister 16 and the wound dressing 20, whereas if a dressing with no absorption capacity or little absorption capacity is used, most or all of the exudate from the wound site 27 may be collected in the canister 16. A suitable filter member (not shown) is generally arranged between the canister 16 and the source of negative pressure 14, e.g., at the outlet port 29 of the canister 16 to ensure that no liquid is allowed to pass to the source of negative pressure 14 from the canister 16. In some embodiments, the filter member is arranged within the canister 14 proximal to the outlet port 29 of the canister.

The control circuitry 11 is further configured to control an operation of the electronically controllable valve 40 so to release negative pressure from the wound site via the second fluid flow path (or "air lumen") 41. In other words, the control circuitry 11 is configured to open the electronically controllable valve 40 so to introduce fluid (e.g. air) to the wound site 27 when there is a sub-atmospheric pressure under the wound cover 22. This operation may also be referred to as "flushing", "venting", "aerating", or "purging". Since the wound treatment system 1 depicted in Fig. 1 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1. Therefore, in order to be able to draw desired amounts of wound exudate from the wound site 27, a fluid, such as e.g. air from the ambient atmosphere, is controllably introduced at defined time intervals and/or in response to pressure measurements by opening the electronically controllable valve 40. In general, once enough fluid has been introduced (e.g. in response to a negative pressure within the first and/or second fluid flow path reaching a lower negative pressure threshold), the control circuitry 11 is configured to close the electronically controllable valve 40 and to activate the source of negative pressure 14. However, in some embodiments, the negative pressure source 14 may be active for at least some of the duration that the electronically controllable valve 40 is open.

The ability to controllably "flush" the system 1 by injecting air via an electronically controllable valve 40 may provide the advantage of longer pressure regulation cycles (may also be referred to as NPWT cycle), which reduces the on-time for the source of negative pressure 14 and therefore reduces the energy consumption of the apparatus 10.

The apparatus 10 may further comprise an air filter (not shown) arranged in the second fluid flow path in order to limit the airflow when the electronically controllable valve 40 is open. In some embodiments, the air filter comprises a hydrophobic and porous material, where the size of the pores is configured to allow for a flow in the range of 50-120 ml/s, such as 60-100 ml/s when the valve 40 is opened after negative pressure has been established under the wound cover 20. The pore size of the filter is preferably measured in a non-compressed state. In some embodiments, the air filter comprises polyethylene or sintered polyethylene. The air filter may also serve to reduce the risk of contaminants entering the wound site 27 when the valve 40 is opened (i.e., during "flushing"). The air filter may be arranged in the tube 41 that at least partly defines the second fluid flow path. The air filter may be arranged upstream of the valve 40 (i.e., between the valve 40 and the inlet of ambient air), or downstream of the valve 40 such as between the valve and the pressure sensor 15a.

Further, the apparatus 10 may be provided with a blockage-detection functionality in order to alert a user (i.e., a patient or a caregiver) of the apparatus 10 of the existence of a blockage condition in the wound treatment system 1. A blockage condition may be caused by an external impact such as a patient lying on a tube 21, 41 or a patient bending the tubing 41, 42. A blockage condition may also be caused by substances which are contained in the fluids sucked away from the wound (such as proteins) that may clog the tubing 21, 41. Similarly, the apparatus 10 may be provided with a leakage-detection functionality or order to alert a user (i.e., a patient or a caregiver) of the apparatus 10 of the existence of a leakage condition in the wound treatment system 1. A leakage condition may be caused by rips or tears in the wound cover 22, detachment of the wound cover 22 from the skin of the patient, improper connections between the tubing 21, 41 and various inlets or outlets, and so forth. A leakage condition may be understood as "excessive leakage" meaning that the apparatus 10 is struggling with establishing or maintaining a set negative pressure level or negative pressure range at the wound site 27. The leakage condition may be for example be detected based on pressure measurements, number of pump 14 activations over a set time period, or a power consumption of the pump 14 over a set time period.

Further, in some embodiments, the apparatus 10 further comprises a communication interface 56 having at least one antenna and at least one transceiver operatively connected to the at least one antenna. Moreover, the transceiver may be operatively connected to the control circuitry 11, and the control circuitry 11 may be configured to transmit and receive signals to and from an external device 50 via the transceiver and antenna. The communication interface 56 may comprise suitable components (e.g. transceivers, antennas, filters, power amplifiers, etc.) with suitable communication protocols (e.g., Wi-Fi, Bluetooth, Bluetooth Low Energy (BLE), Zigbee, or the like) in order to establish a connection with an external device 50 and to transmit and receive wireless signals to and from the external device 50. Accordingly, the communication interface 56 is configured to transmit and receive wireless signals to and from the external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network or cellular or mobile phone communications transceivers. In another example, the communication interface 56 may include a short-range wireless transmitter (e.g., a Bluetooth wireless transmitter, etc.) for communicating via a short-range wireless communication transceiver. The control circuitry 11 may be configured to send one or more alarm signals or notifications indicative of a condition of the apparatus 10 (e.g., blockage alarm, leakage alarm, low battery charge alarm) to the external device 50 via the one or more antennas.

The external device may be an external handheld device 50, such as a mobile phone (e.g. a smart phone) operatively connected to the apparatus 10. Moreover, the handheld device 50 preferably comprises corresponding hardware and software capabilities to establish a wireless communication link with the apparatus 10 and to transmit and receive signals to and from the apparatus 10.

In some embodiments, the apparatus 10 comprises a user interface. The user interface may for example comprise the button 31 used to start/pause/stop the negative pressure source 14 mentioned in the foregoing. The user interface may further comprise one or more LEDs 62 configured to emit a light signal to a user of the apparatus 10. For example, the LEDs 62 may be used to indicate a presence of a blockage condition, a leakage condition, and/or a low battery charge condition. The LEDs 62 may be arranged on the housing 19 of the apparatus and operatively connected to the control circuitry 11.

It should be noted that even though the terms "exudate lumen" and "air lumen" are sometimes mentioned instead of "first fluid flow path" and "second fluid flow path", this should not be construed as that the first and second fluid flow paths are limited to solely consist of the tubes 21, 22 between the connector 25 and the apparatus 10 of the wound treatment system 1. For example, as depicted in Fig. 1 the first fluid flow path may comprise the first the connector 25, the first tubing 21, the canister 16, as well as any smaller tubing/manifolds/connectors between the canister 16 and the inlet of the source of negative pressure 14. Instead, the terms "exudate lumen" and "air lumen" are mainly used to increase readability, and could be read as "first fluid flow path" and "second fluid flow path".

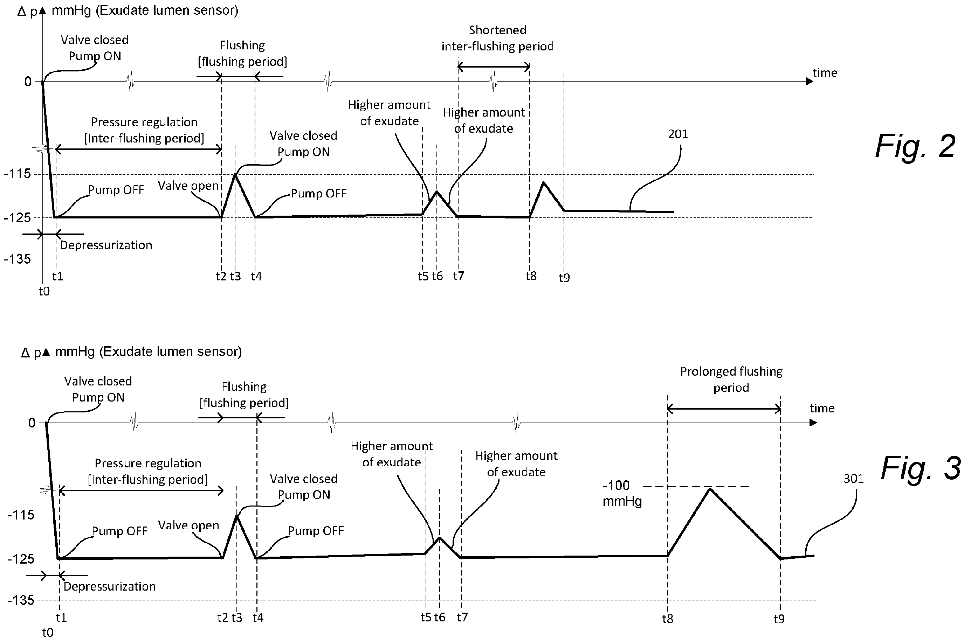

In some embodiments, during use, the control circuitry 11 is configured to regulate the negative pressure provided to the wound site over a plurality of pressure regulation cycles. In general, a pressure regulation cycle may be understood as a time period comprising at least a "pressure regulation period" and a "flushing period" (see e.g., Figs. 2-11). The "pressure regulation period" is herein referred to as "inter-flushing period" that defines a time between two consecutive flushing periods (and eventually the time between the end of the initial depressurization from atmospheric to therapeutic pressure and the first flushing period). During the "inter-flushing period" the control circuitry 11 is configured to operate the negative pressure source 14 so to maintain a level of negative pressure to be within a suitable negative pressure range as exemplified above, i.e., the apparatus 10 is operated in a "pressure regulation mode". In other words, during the "inter-flushing period" the electronically controllable valve 40 is closed and the source of negative pressure 14 is operated to maintain a desired level of negative pressure at the wound site 27. The desired level of negative pressure may for example comprise a negative pressure range. During the "flushing period" the control circuitry 11 is configured to open the electronically controllable valve 40 so to introduce fluid (e.g., air from the ambient atmosphere) to the wound site 27, close the valve 40, and activate the source of negative pressure 14 to draw wound exudate from the wound site and re-establish the negative pressure level, i.e., the apparatus 10 is operated in a "flushing mode". Accordingly, the flushing period causes a temporary decrease in negative pressure at the wound site 27 (i.e., a temporary increase in absolute/gauge pressure).

Conventionally, the duration of an inter-flushing period and a flushing period, and accordingly the duration of a pressure regulation cycle are fixed, or in other words, time-controlled. For example, the apparatus 10 may be configured to execute a flushing sequence every 30 minutes or every hour for the entire therapy duration. However, this may be sub-optimal, both from a therapeutic perspective and an energy consumption perspective. For example, for high exuding wounds, it may be desirable to execute flushing sequences more frequently as compared to low exuding wounds in order to avoid accumulation of exudates at the wound site 27. Even though this can be accounted for by the caregiver when setting up the apparatus 10 prior to use, it is difficult to predict the amount of exudate that will be generated by the wound over the entire duration of the therapy, which may be several weeks.

To this end, some embodiments herein propose an apparatus 10 for providing negative pressure to a wound site that is capable of dynamically controlling the duration of the inter-flushing periods and the flushing periods based on estimated current "need" for "exudate transport". This provides an advantage of having an apparatus 10 that is capable of adaptively controlling the frequency of flushing sequences during use to ensure adequate exudate removal from the wound site without unnecessarily increasing overall energy consumption of the apparatus 10. In other words, an advantage of some embodiments is that the wound site 27 is drained of wound exudate frequently enough to improve the efficiency of the therapy without excessive energy consumption. Another advantage of some embodiments is that there is no need for complex configurations for the timing of the flushing sequences that have to be manually set beforehand based on an assessment of the wound as the apparatus 10 is capable of doing so automatically during run-time. Thus, user-friendliness may be improved.

Accordingly, the control circuitry 11 is configured to estimate an amount of exudate to be moved from the wound site via a portion of the first fluid flow path to the canister, and control a duration of the flushing period and/or a duration of the inter-flushing period based on the estimated amount of exudate. As mentioned, the flushing period defines a time period during which the electronically controllable valve is opened and the source of negative pressure is activated for transporting fluid from the wound site to the canister, and the inter-flushing period defines a time between two consecutive flushing periods.

In the other words, the flushing period defines the set duration of time for the control circuitry 11 to execute a flushing sequence, while the inter-flushing period defines the set duration of time between two consecutive flush sequences. Since, the therapy may start with an inter-flushing period following an initial depressurization, the inter-flushing period may also define the set duration of time between the end of the initial depressurization and the first flushing period. However, the therapy may start with a flushing period following the initial depressurization, and in that case the inter-flushing period defines the time between two consecutive flushing sequences.

The term "amount of exudate to be moved from the wound site via a portion of the first fluid flow path to the canister" may be understood as "an amount of exudate in need of transportation from the wound site via a portion of the first fluid flow path to the canister", "an amount of exudate present in the fluid circuit of the wound treatment system" (where the fluid circuit includes at least the first fluid flow path and wound enclosure), "an amount of exudate present in the first fluid flow path", "an amount of exudate present in the first and second fluid flow paths", or "an amount of non-absorbed exudate present in the wound treatment system".

Similarly, the term "an amount of exudate" does not necessarily refer to a quantified value, such as for example X ml, but may alternatively refer to a relative value such as, high, medium, low, or none. Thus, in some embodiments, the estimation of an amount of exudate to be moved from the wound site may comprise estimating whether there is a high, medium, or low amount of exudate to be moved from the wound site. Moreover, the term "estimating an amount" may be construed as "determining an amount", "calculating an amount", "deriving an amount", or "approximating an amount".

Various example embodiments describing how the amount of exudate may be estimated are provided below in reference to the schematic graphs of negative pressure over time, Δp(t), illustrated in Figs. 2-11. It should be noted that the pressure curves are schematic and not necessarily to scale, some parts have been exaggerated (e.g., the duration of the flushing period relative to the pressure regulation period, steepness of curves, etc.) to increase readability.

Each of Figs. 2-11 illustrate a "depressurization stage" between time to and t1, a first "inter-flushing period" between time t1 and t2, a first "flushing period" between time t2 and t4, a second "inter-flushing period" between time t4 and t5, and a second "flushing period" between time t5 and t7. During the depressurization stage the apparatus 10 undergoes a first depressurization (e.g., initial activation of the apparatus 10) to establish a negative pressure at the wound site 27 by activating the source of negative pressure 14 while keeping the valve 40 closed. The source of negative pressure 14 may then be turned off in response to some set negative pressure value being reached (-125 mmHg in the depicted embodiment).

Subsequently, the apparatus 10 enters an "inter-flushing period", which may also be referred to as a "pressure regulation period", "therapy regulation period", or "negative pressure maintenance period" where the apparatus 10 is intended to maintain a certain negative pressure at the wound site 27 for a set duration of time. The control circuitry 11 may be configured to monitor the pressure level output by the exudate lumen pressure sensor 15a and/or the air lumen pressure sensor 15b and activate the source of negative pressure as needed (e.g., due to "small" parasitic leakages) to maintain the certain negative pressure at the wound site 27. The duration of the first "inter-flushing period" may be predefined and extends from time t1 to t2 in the depicted embodiments. Next, the apparatus enters a flushing period, at time t2, where the electronically controllable valve 40 is opened for a duration of time (t2 to t3) whereupon the valve 40 is closed and the source of negative pressure 14 is turned on, at time t3, to increase the negative pressure at the wound site 27. It should be noted that the source of negative pressure 14 may be turned on for some duration or the entire duration of the flushing period (i.e., while the valve 40 is open). Then, after expiry of a set time period (t3 to t4) the source of negative pressure 14 is turned off. This completes a pressure regulation cycle for the apparatus 10.

It should be noted that the flushing sequence may also be pressure-controlled, meaning that the valve 40 is opened at t2 and kept open until a set pressure value is reached (e.g., -115 mmHg) whereupon the valve is closed 40 and the pump 14 is activated until another set pressure value is reached (e.g., -125 mmHg). In those cases, the duration of the flushing period may be controlled by appropriately adjusting the pressure thresholds that trigger valve closing, pump activation, and pump deactivation. Alternatively, the flushing sequence may be time-and-pressure-controlled, meaning that the valve 40 is opened at t2 and kept open for a duration of time (t2 to t3) or until some set pressure value is reached (e.g., -115 mmHg), which ever occurs first. Then, the valve 40 is closed and the source of negative pressure 14 is turned on for a set duration of time (t3 to t4) or until some pressure value is reached (e.g., -125 mmHg). The apparatus 10 then enters a second inter-flushing period from time t4 to t5. The second inter-flushing period has the same duration as the first inter-flushing period (t1 to t2 = t4 to t5) as there has yet been no indication of increased or decreased amounts to be moved from the wound site via a portion of the first fluid flow path to the canister.

Turning now to Fig. 2 and Fig. 3 depicting two schematic graphs 201, 301 of negative pressure over time, Δp(t), where an increased amount of exudate is detected based on a pressure change rate as output by an exudate lumen pressure sensor 15a, in accordance with some embodiments. As a result of this, a subsequent inter-flushing period may be shortened as illustrated in Fig. 2, or a subsequent flushing period may be prolonged as illustrated in Fig. 3.

In more detail, the control circuitry 11 may be configured to estimate the amount of exudate to be moved by comparing a pressure change rate as output by the first pressure sensor 15a while the source of negative pressure 14 is active with a previous pressure change rate as output by the first pressure sensor 15a while the source of negative pressure 14 was active during a preceding time period, and estimating the amount of exudate based on the comparison between the pressure change rate and the previous pressure change rate. In other words, the amount of exudate to be moved may be estimated by comparing the pressure change rate as output by the first pressure sensor 15a between time t6 and t7 with a "historical pressure change rate" as output by the first pressure sensor 15a, such as for example the pressure change rate between time t3 and t4 or t0 and t1. The pressure change rate measurements may be step or impulse responses after pump 14 activation, or pressure change rate measurements over a longer time period (e.g., between time t0-t1, t3-t4, and t6-t7).

In some embodiments, the "historical pressure change rate" may be the pressure change rate output as output by the first pressure sensor 15a while the source of negative pressure 14 was active during the preceding flushing period. However, in some embodiments, the "historical pressure change rate" may be an averaged value of multiple pressure change rate samples as output by the first pressure sensor 15a while the source of negative pressure 14 was active during a preceding time period (e.g., during the last 6 hours, during the last 24 hours, since start of therapy, and so forth).

In some embodiments, the control circuitry 11 may be configured to determine that there is an increased or high amount of exudate to be moved from the wound site 27 in response to the pressure change rate being lower than a previous pressure change rate as output by the first pressure sensor 15a while the source of negative pressure 14 was active during a preceding time period. Then, in response to determining that there is an increased or high amount of exudate to be moved from the wound site 27, the control circuitry 11 may be configured to shorten one or more upcoming inter-flushing periods (Fig. 2) and/or prolong one or more upcoming flushing periods (Fig. 3).

By shortening the inter-flushing period, the flushing sequences are executed more frequently and more exudate can be transported from the wound site 27 to the canister 16. Similarly, by prolonging the flushing period, more air is circulated in the system and more wound exudate can be transported from the wound site 27 to the canister 16.

In some embodiments, the control circuitry 11 is configured to estimate the amount of exudate by comparing a pressure change rate as output by the first pressure sensor 15a while the source of negative pressure 14 is active with a set pressure change rate value (e.g., predefined pressure change rate value), and estimating the amount of exudate based on the comparison between the pressure change rate and the set pressure change rate value. In other words, instead of using a "historical pressure change rate value", one can use a set/predefined pressure change rate in the comparison. The set/predefined pressure change rate value may for example be determined from test data and chosen to define a pressure change rate where the amount of exudate to be moved from the wound site 27 to the canister 16 is at a level where a "standard setting" for the durations of the flushing period and inter-flushing period is adequate.

Even though the adaptation of the inter-flushing period and the flushing period as illustrated as separate "reactions" to the estimation that there is an increased or high amount of exudate to be moved from the wound site 27, they may be combined. In other words, based on the estimated amount of exudate one can either control the duration of the flushing period, the duration of the inter-flushing period, or both. The separation in Fig. 2 and Fig. 3 is mainly performed to facilitate readability.

Furthermore, in some embodiments, the control circuitry 11 is configured to estimate the amount of exudate by comparing a pressure change rate as output by the first pressure sensor 15a after opening the electronically controllable valve 40 with a set pressure change rate value, and estimating the amount of exudate based on the comparison between this pressure change rate and the set pressure change rate value. In other words, rather than monitoring the pressure change rate between time t6 and t7, one instead monitors the pressure change rate between time t5 and t6. As before, the comparison may be done in view of a "historical" pressure change rate value (e.g., the pressure change rate value between time t2 and t3) rather than the set or otherwise predefined pressure change rate value.

In some embodiments, the control circuitry 11 may be configured to determine that there is an increased or high amount of exudate to be moved from the wound site 27 in response to the pressure change rate, after valve 40 opening (e.g., between t5 and t6), being lower than the set/historical pressure change rate value. Then, in response to determining that there is an increased or high amount of exudate to be moved from the wound site 27, the control circuitry 11 may be configured to shorten one or more upcoming inter-flushing periods (Fig. 2) and/or prolong one or more upcoming flushing periods (Fig. 3).

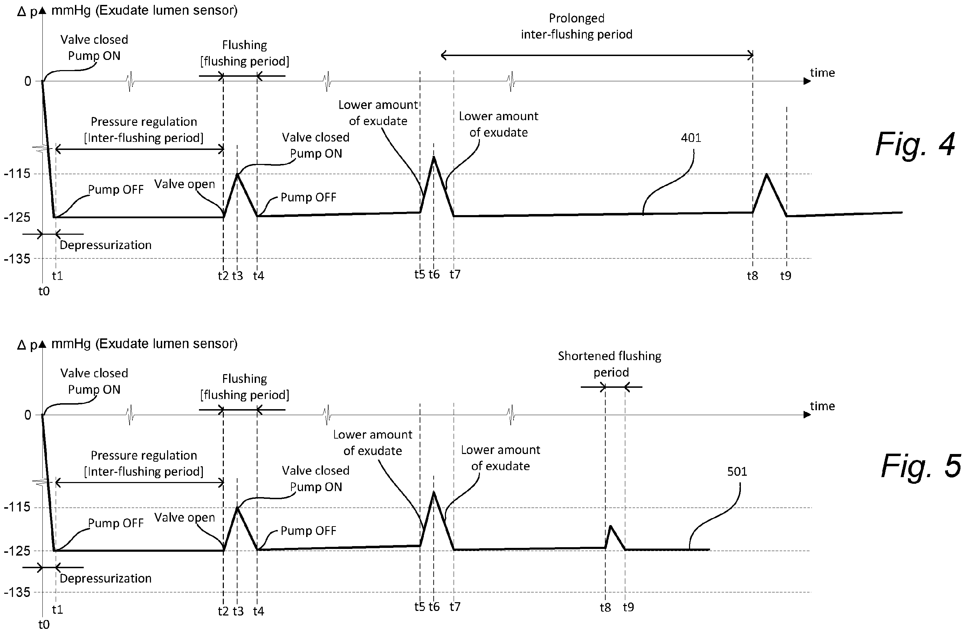

Turning now to Fig. 4 and Fig. 5, which depict schematic graphs 401, 501 of negative pressure over time, Δp(t), where a reduced or low amount of exudate is estimated based on a pressure change rate as output by an exudate lumen pressure sensor 15a, in accordance with some embodiments. As a result of this, a subsequent inter-flushing period may be prolonged as illustrated in Fig. 4, or a subsequent flushing period may be shortened as illustrated in Fig. 5. In other words, as compared to Fig. 2 and Fig. 3, the graphs 401, 501 of Fig. 4 and Fig. 5 illustrate the opposite scenario where pressure change rate measurements indicate a reduced or low amount of exudate to be moved to the canister 16 from the wound site to the canister rather than an increased or high amount of exudate to be moved to the canister 16 from the wound site 27. The working principles are analogous and will for the sake of brevity and conciseness not be repeated.

Thus, in some embodiments, the control circuitry 11 may be configured to determine that there is a decreased or low amount of exudate to be moved from the wound site 27 in response to the pressure change rate being higher than a set pressure change rate or previous pressure change rate as output by the first pressure sensor 15a while the source of negative pressure 14 was active during a preceding time period. Then, in response to determining that there is a decreased or low amount of exudate to be moved from the wound site 27, the control circuitry 11 may be configured to prolong one or more upcoming inter-flushing periods (Fig. 4) and/or shorten one or more upcoming flushing periods (Fig. 5).

Moreover, in some embodiments, the control circuitry 11 may be configured to determine that there is a decreased or low amount of exudate to be moved from the wound site 27 in response to the pressure change rate, after valve 40 opening (e.g., between t5 and t6), being lower than the set/historical pressure change rate value. Then, in response to determining that there is a decreased or low amount of exudate to be moved from the wound site 27, the control circuitry 11 may be configured to prolong one or more upcoming inter-flushing periods (Fig. 4) and/or shorten one or more upcoming flushing periods (Fig. 5).

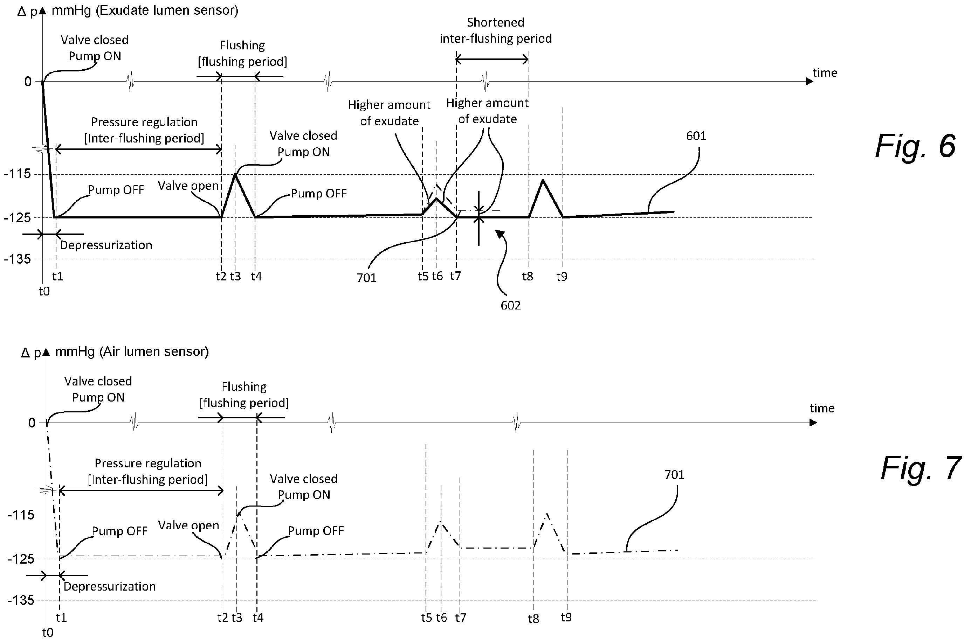

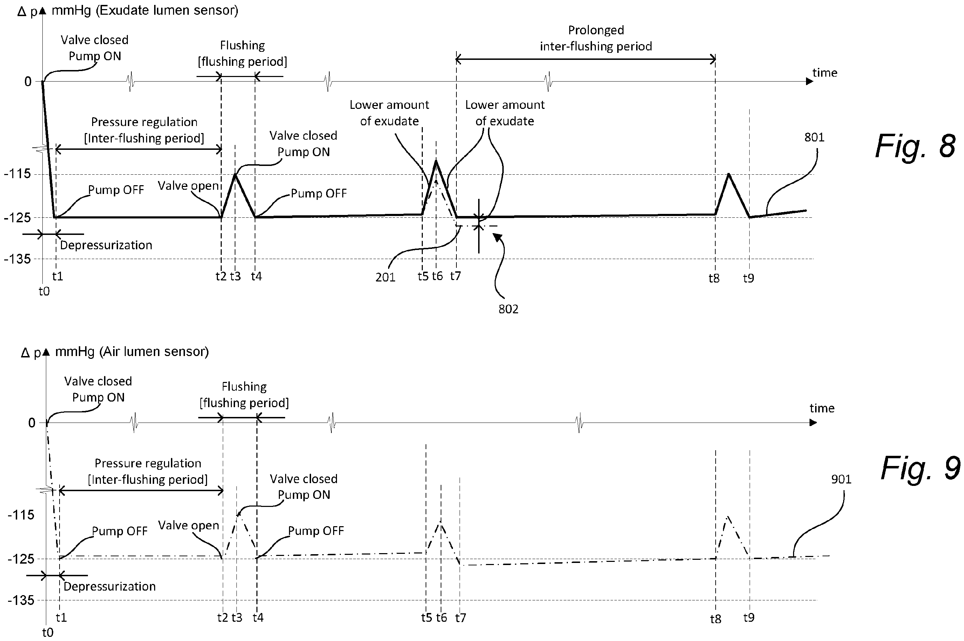

Moving on, in some embodiments, the apparatus 10 further comprises a second pressure sensor 15b configured to monitor a pressure level in the second fluid flow path. Accordingly, the control circuitry 11 may be operatively connected to the second pressure sensor 15b and configured to estimate the amount of exudate by comparing a first pressure level as output by the first pressure sensor 15a after the source of negative pressure 14 has been active for a duration of time with a second pressure level as output by the second pressure sensor 15b after the source of negative pressure 14 has been active for the duration of time, and estimating the amount of exudate based on the comparison between the first pressure level and the second pressure level. In other words, one compares the pressure level in the air lumen with the pressure level in the exudate lumen in order to conclude whether there are low or high amounts of exudate in the fluid circuit of the system 1. This is for example indicated by reference numeral 602 and 802 in Figs. 6 and 8, respectively. It should be noted that the comparison in pressure levels as output by each of the two pressure sensors 15a, 15b may also be done after the valve 40 has been opened instead of, or in addition to, making the comparison after the source of negative pressure 14 has been active for a duration of time. The term "after the source of negative pressure has been active for a duration of time" or "after the valve has been opened" are to be interpreted broadly and the timing may differ depending on specific applications and one can measure the pressure levels upon expiry of a suitably configured counter (e.g., after 1s, after 2s, after 3s, after 5s, etc.).

Thus, in contrast to the embodiments discussed in the foregoing with reference to Figs. 2-5, where the output from a single exudate lumen pressure sensor 15a was used to estimate the amount of exudate, one can utilize a difference between the outputs of the exudate and air lumen pressure sensors to estimate the amount of exudate. This is depicted in Fig. 6 - Fig. 9, where Fig. 6 and 8 are schematic graphs 601, 801 of negative pressure over time, Δp(t), as output by the exudate lumen pressure sensor 15a, while Figs. 7 and 9 are schematic graphs 701, 901 of negative pressure over time, Δp(t), as output by the air lumen pressure sensor 15b. Moreover, in Fig. 6 and 8 there is a small segment of the air lumen pressure curve 701, 901 interposed for comparison in connection with the second flushing period.

Analogous to the embodiments depicted in Figs. 2-5 discussed in the foregoing, Figs. 6 and 7 show a scenario where an increased or high amount of exudate is detected, while Figs. 8 and 9 show a scenario where a decreased or low amount of exudate is detected.

Accordingly, in some embodiments, the control circuitry 11 may be configured to determine that there is an increased or high amount of exudate to be moved from the wound site 27 in response to the first pressure level as output by the first pressure sensor 15a while the source of negative pressure 14 is active being lower (i.e., higher negative pressure) than the second pressure level as output by the second pressure 15b after the source of negative pressure has been active for the duration of time (e.g., after t7). Then, in response to determining that there is an increased or high amount of exudate to be moved from the wound site 27, the control circuitry 11 may be configured to shorten one or more upcoming inter-flushing periods (Fig. 6) and/or prolong one or more upcoming flushing periods (not shown). As before, as an alternative or addition to comparing the pressure levels after pump 14 activation, one could compare the pressure levels after valve 40 opening.

As an alternative or addition to relying on the difference in pressure level, one may look at the difference in pressure change rate as output by the pressure sensors 15a, 15b. In more detail, in some embodiments, the control circuitry 11 is configured to estimate the amount of exudate by comparing a first pressure change rate as output by the first pressure sensor 15a while the source of negative pressure is active with a second pressure change rate as output by the second pressure sensor 15b while the source of negative pressure is active, and estimating the amount of exudate based on a comparison between the first pressure change rate and the second pressure change rate. In other words, the amount of exudate to be moved may be estimated by comparing the pressure change rate as output by the first pressure sensor 15a between time t6 and t7 with the pressure change rate as output by the second pressure sensor 15b during the same time period.

Similar as before the comparison in pressure change rates as output by each of the two pressure sensors 15a, 15b may also be done after the valve 40 has been opened as an alternative or addition to making the comparison after the source of negative pressure 14 has been active for a duration of time. Thus, one could monitor and compare the pressure change rates between time t5 and t6 and not only the pressure change rates between time t6 and t7.

Accordingly, in some embodiments, the control circuitry 11 may be configured to determine that there is an increased or high amount of exudate to be moved from the wound site 27 in response to the first pressure change rate as output by the first pressure sensor 15a while the source of negative pressure is active (e.g., between t6 and t7) being lower (i.e., slower decrease in pressure) than the second pressure change rate as output by the second pressure 15b while the source of negative pressure is active. Then, in response to determining that there is an increased or high amount of exudate to be moved from the wound site 27, the control circuitry 11 may be configured to shorten one or more upcoming inter-flushing periods (Fig. 6) and/or prolong one or more upcoming flushing periods (not shown). As before, as an alternative or addition to comparing the pressure change rates after pump 14 activation, one could compare the pressure change rates after valve 40 opening.

Turning now to Fig. 8 and Fig. 9, which depict schematic graphs 801, 901 of negative pressure over time, Δp(t), where a reduced or low amount of exudate is detected, in accordance with some embodiments. Just as for the above discussed scenarios in reference to Figs. 6 and 7, the amount of exudate is estimated based on a difference in pressure levels in the exudate and air lumens and/or based on a difference in pressure change rates as output by the two pressure sensors 15a, 15b. As a result of this, a subsequent inter-flushing period may be prolonged as illustrated in Fig. 4, or a subsequent flushing period may be shortened (not shown). In other words, as compared to Fig. 6 and Fig. 7, the graphs 801, 901 of Fig. 4 and Fig. 5 illustrate the opposite scenario where pressure level measurements or pressure change rate measurements indicate a reduced or low amount of exudate to be moved to the canister 16 from the wound site to the canister rather than an increased or high amount of exudate to be moved to the canister 16 from the wound site 27. The working principles are analogous and will for the sake of brevity and conciseness not be repeated.

Accordingly, in some embodiments, the control circuitry 11 may be configured to determine that there is an reduced or low amount of exudate to be moved from the wound site 27 in response to the first pressure level as output by the first pressure sensor 15a while the source of negative pressure 14 is active being higher (i.e., lower negative pressure) than the second pressure level as output by the second pressure 15b after the source of negative pressure has been active for the duration of time (e.g., after t7). Then, in response to determining that there is a reduced or low amount of exudate to be moved from the wound site 27, the control circuitry 11 may be configured to prolong one or more upcoming inter-flushing periods (Fig. 8) and/or shorten one or more upcoming flushing periods (not shown). As before, as an alternative or addition to comparing the pressure levels after pump 14 activation, one could compare the pressure levels after valve 40 opening.

Moreover, , the control circuitry 11 may be configured to determine that there is an reduced or low amount of exudate to be moved from the wound site 27 in response to the first pressure change rate as output by the first pressure sensor 15a while the source of negative pressure is active (e.g., between t6 and t7) being higher (i.e., faster decrease in pressure) than the second pressure change rate as output by the second pressure 15b while the source of negative pressure is active. Then, in response to determining that there is a reduced or low amount of exudate to be moved from the wound site 27, the control circuitry 11 may be configured to prolong one or more upcoming inter-flushing periods (Fig. 8) and/or shorten one or more upcoming flushing periods (not shown). As before, as an alternative or addition to comparing the pressure change rates after pump 14 activation, one could compare the pressure change rates after valve 40 opening.

Furthermore, the estimations of the amount of exudate to be moved from the wound site 27 via a portion of the first fluid flow path to the canister 14 as low, normal, or high or even as discrete values (e.g., X ml) may be done using any one of the techniques mentioned in the foregoing by applying specific thresholds. These thresholds may either be derived from simulations or from a live test-setup. For example, in reference to the exudate estimate using a difference in pressure level between the exudate lumen pressure sensor 15a and the air lumen pressure sensor 15b, one can arrange a test-setup with a wound treatment system 1 as disclosed herein where one applies or injects different amounts of liquid the under the wound cover 22 or in the fluid circuit in general, and then run a testing protocol to connect differences in pressure level with different amounts of liquid. In other words, one could measure the pressure level differences for different amounts of injected liquid in the test-setup. These pressure level differences may then serve as thresholds or "expected values", where each value is associated with a specific amount of liquid/exudate. The thresholds may for example be stored in a look-up table or any other suitable data format in the memory 12 of the apparatus 10. Then during use, the pressure level difference is compared to these thresholds or expected values in order to derive or approximate the amount of exudate to be moved to the canister 16.

It should be noted that even though the operation of estimating amount of exudate to be moved from the wound site 27 via a portion of the first fluid flow path to the canister 16 is performed during a flushing sequence in Figs. 2-9, it may be performed at any time during a pressure regulation cycle. In more detail, the estimation may for example be performed at certain time intervals independently whether the apparatus 10 is in a pressure regulation mode or a flushing mode by for example activating the source of negative pressure 14 or opening the valve 40 for a brief moment in time. The pressure data (pressure change rate or pressure level) is then checked after pump 14 activation or valve 40 opening, e.g., as a step response or impulse response. However, an advantage of doing it concurrently or in combination with the flushing sequence is that it alleviates the need for a separate "exudate estimation process" and therefore removes additional pump activations that increase the energy consumption of the apparatus 10.

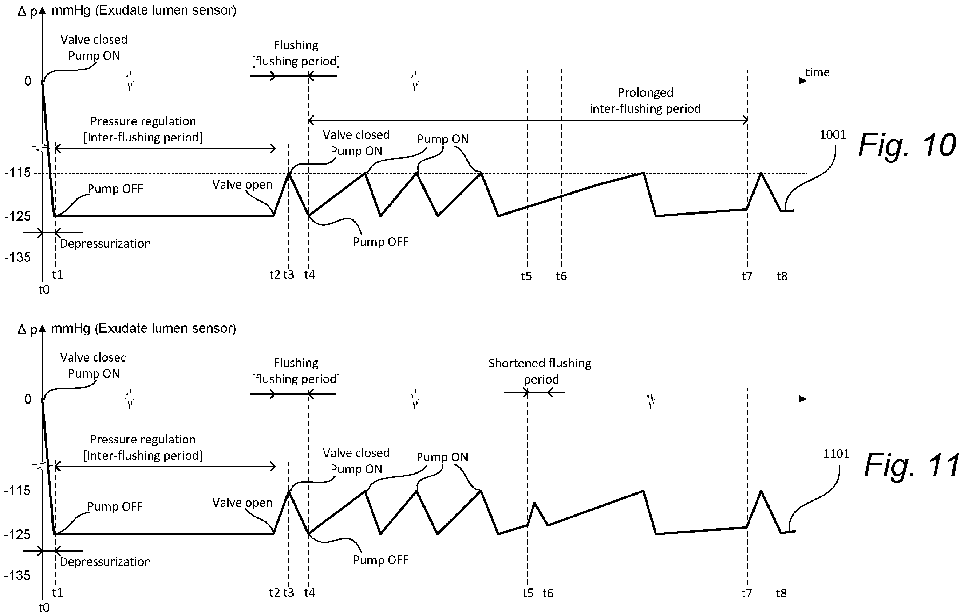

The prolonging of the flushing period as for example illustrated in Fig. 3 may be achieved by changing the threshold for when the valve 40 is to be closed in the prolonged flushing sequence to a higher pressure value (i.e., lower negative pressure value) as compared to a nominal flushing sequence. For example, in a nominal flushing sequence the valve 40 is opened and then closed upon reaching some set pressure value (e.g., -115 mmHg), while in the prolonged flushing sequence the valve 40 is opened and then closed upon reaching a different, higher, pressure value (e.g., -100 mmHg). Analogously, the shortening of the flushing period as for example illustrated in Fig. 5 and Fig. 11 may be achieved by changing the threshold for when the valve 40 to a lower pressure value (i.e., higher negative pressure value) as compared to a nominal flushing sequence. For example, in a nominal flushing sequence the valve 40 is opened and then closed upon reaching some set pressure value (e.g., -115 mmHg), while in the prolonged flushing sequence the valve 40 is opened and then closed upon reaching a different, lower, pressure value (e.g., -120 mmHg).

Turning now to Fig. 10 and Fig. 11, which depict schematic graphs 1001, 1101 of negative pressure over time, Δp(t), where a reduced or low amount of exudate is estimated based on a number of pump 14 activations during an inter-flushing period, in accordance with some embodiments. The pump activations may for example be monitored and a count of activations may be stored in the memory 12.

in some embodiments, the control circuitry 11 is configured to estimate the amount of exudate by counting a number of activations of the source of negative pressure 14 during at least a portion of an inter-flushing period, comparing the number of counted activations of the source of negative pressure 14 with a set value (e.g., 5, 10, or 15 depending on the configured duration of the inter-flushing period), and estimating the amount of exudate based on the comparison between the number of counted activations and the set value. However, in some embodiments the comparison may be made between the number of counted activations and a historical value of pump activations, such as for example pump activations in a preceding pressure regulation cycle, or the average number of pump activations in N preceding pressure regulation cycles, where N is a positive integer > 1.

Moving on, the control circuitry may be configured to determine that there is a reduced or low amount of exudate to be moved from the wound site 27 in response to the number counted activations of the source of negative pressure 14 during at least a portion of an inter-flushing period (e.g., between t4 and t5) exceeding a set value or historical value. Then, in response to determining that there is a decreased or low amount of exudate to be moved from the wound site 27, the control circuitry 11 may be configured to prolong one or more upcoming inter-flushing periods (Fig. 10) and/or shorten one or more upcoming flushing periods (Fig. 11).

The increased amount of pump 14 activations during the second inter-flushing period may for example be caused due to an "elevated leakage scenario" that may be understood as that the wound treatment system 1 has some unwanted leakage ("parasitic leakage"). The unwanted leakage may for example be due to improperly applied wound cover 22 and/or leaky connections between the tubing and other components. Thus, if a sufficiently high number of pump 14 activations have taken place during an inter-flushing period it can be assumed that any wound exudate in the fluid circuit of the system 1 has most likely already been transported to the canister 16 as enough air has been added to the system to move the exudate. Thus, executing the planned flushing sequence may be unnecessary as it is unlikely that there is any significant amount of exudate that needs to be transported to the canister 16. The set value that the counted activations are compared to may for example be set based on system testing where a controlled leakage is introduced and the number of activations needed to transport some defined amount of exudate is counted and used as a threshold. The set value may be different from different wound treatment systems 1 as they may have different system volumes.

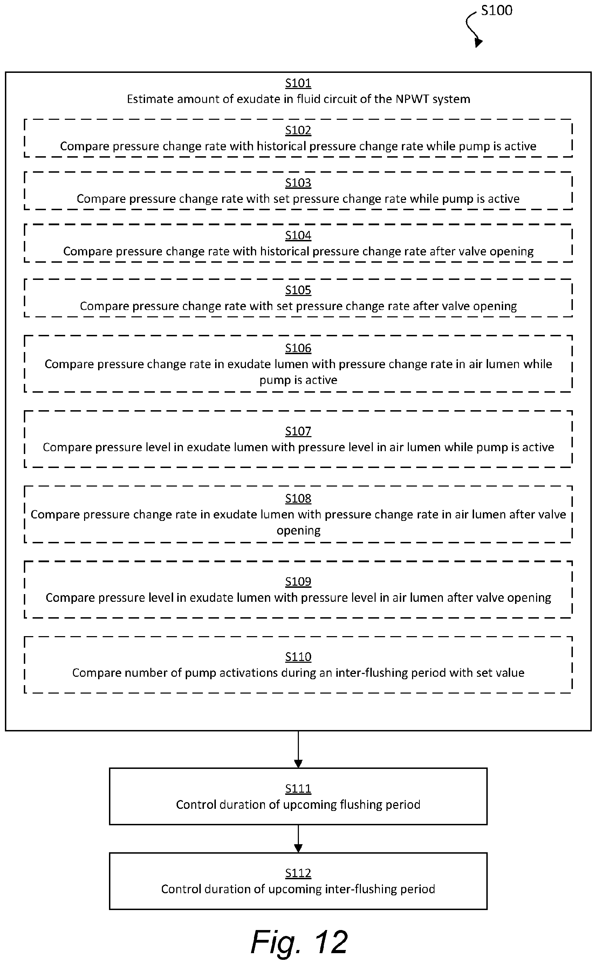

Fig. 12 is a schematic flowchart representation of a method S100 for operating an apparatus 10 for providing negative pressure to a wound site 27 in accordance with any one of the embodiments disclosed herein. The apparatus 10 comprises a source of negative pressure 14 configured to provide negative pressure via a first fluid flow path from an inlet of the source of negative pressure 14 to a wound site 27, a canister 16 for receiving fluids from the wound site 27, and an electronically controllable valve 40 configured to release negative pressure from the wound site 27 via a second fluid flow path. The apparatus 10 further comprises a pressure sensor configured to monitor/measure/detect a pressure level in the first fluid flow path. The method S100 comprises estimating S101 an amount of exudate to be moved from the wound site via a portion of the first fluid flow path to the canister, and controlling S111 a duration of a flushing period and/or controlling S112 a duration of an inter-flushing period based on the estimated amount of exudate. As before, the flushing period defines a time period during which the electronically controllable valve 40 is opened and the source of negative pressure 14 is activated for transporting fluid from the wound site 27 to the canister 16, and the inter-flushing period defines a time between two consecutive flushing periods.

In some embodiments, the estimation S101 of the amount of exudate comprises comparing S102 a pressure change rate as detected in the first fluid flow path while the source of negative pressure is active with a previous pressure change rate as detected in the first fluid flow path while the source of negative pressure was active during a preceding time period, and estimating the amount of exudate based on the comparison between the pressure change rate and the previous pressure change rate. In some embodiments, the estimation S101 of the amount of exudate comprises comparing S103 a pressure change rate as detected in the first fluid flow path while the source of negative pressure is active with a set pressure change rate, and estimating the amount of exudate based on the comparison between the pressure change rate and the set pressure change rate. As before, it should be noted that the comparison S104, S105 between the pressure change rate and the historical or set pressure change rate may be done after a valve 40 opening in an analogous manner.

Further, the apparatus 10 may comprise a second pressure sensor configured to monitor a pressure level in the second fluid flow path. Thus, in some embodiments, the estimation S101 of the amount of exudate comprises comparing S106 a first pressure change rate as detected in the first fluid flow path while the source of negative pressure 14 is active with a second pressure change rate as detected in the second fluid flow path while the source of negative pressure 14 is active, and estimating the amount of exudate based on a comparison between the first pressure change rate and the second pressure change rate. As before, one may also compare S107 a first pressure level as detected in the first fluid flow path after the source of negative pressure 14 has been active for a duration of time with a second pressure level as detected in the second fluid flow path after the same time period in order to estimate the amount of exudate.

In some embodiments, the estimation S101 of the amount of exudate comprises comparing S108 a first pressure change rate as detected in the first fluid flow path after opening the electronically controllable valve 40 with a second pressure change rate as detected in the second fluid flow path after opening the electronically controllable valve 40, and estimating the amount of exudate based on a comparison between the first pressure change rate and the second pressure change rate. As before, one may also compare S109 a first pressure level as detected in the first fluid flow path after opening the electronically controllable valve 40 with a second pressure level as detected in the second fluid flow path after opening the electronically controllable valve 40 in order to estimate the amount of exudate.

Further, in some embodiments, the estimation S101 of an amount of exudate to be moved from the wound site 27 via a portion of the first fluid flow path to the canister 16 comprises counting a number of activations of the source of negative pressure 14 during at least a portion of an inter-flushing period, comparing S110 the number of counted activations of the source of negative pressure 14 with a set value, and estimating the amount of exudate based on the comparison between the number of counted activations and the set value. As before, the comparison may be made between the number of counted activations and a historical value of pump activations, such as for example pump activations in a preceding pressure regulation cycle, or the average number of pump activations in N preceding pressure regulation cycles, where N is a positive integer > 1.

Executable instructions for performing these functions are, optionally, included in a non-transitory computer-readable storage medium or other computer program product configured for execution by one or more processors of an apparatus 10 for providing reduced pressure to a site.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments embodiment, there is provided a non-transitory computer-readable storage medium storing one or more programs configured to be executed by one or more processors of an apparatus of a reduced-pressure wound treatment system, the one or more programs comprising instructions for performing the method according to any one of the above-discussed embodiments.

Generally speaking, a computer-accessible medium may include any tangible or non-transitory storage media or memory media such as electronic, magnetic, or optical media coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 11 (associated with the apparatus 10) may be or include any number of hardware components for conducting data or signal processing or for executing computer code stored in memory. The device 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing negative pressure to a wound site (27), the apparatus comprising:
a source of negative pressure (14) configured to provide negative pressure via a first fluid flow path from an inlet of the source of negative pressure to a wound site;
a canister (16) for receiving fluids from the wound site, wherein the canister has a canister outlet (29) adapted to be fluidly coupled to the inlet of the source of negative pressure and a canister inlet (28) adapted to be fluidly coupled to the wound site;
an electronically controllable valve (40) configured to release negative pressure from the wound site via a second fluid flow path;
a first pressure sensor (15a) configured to monitor a pressure level in the first fluid flow path;
control circuitry (11) operatively connected to the source of negative pressure (14), the electronically controllable valve (40), and to the first pressure sensor (15a);
wherein the control circuitry (11) is configured to:
estimate an amount of exudate to be moved from the wound site (27) via a portion of the first fluid flow path to the canister, and
control a duration of a flushing period and/or a duration of an inter-flushing period based on the estimated amount of exudate;
wherein the flushing period defines a time period during which the electronically controllable valve is opened and the source of negative pressure is activated for transporting fluid from the wound site to the canister, and the inter-flushing period defines a time between two consecutive flushing periods.

2. The apparatus (10) according to claim 1, wherein the control circuitry (11) is configured to estimate the amount of exudate by:
comparing a pressure change rate as output by the first pressure sensor (15a) while the source of negative pressure (14) is active with a previous pressure change rate as output by the first pressure sensor (15a) while the source of negative pressure (14) was active during a preceding time period,
estimating the amount of exudate based on the comparison between the pressure change rate and the previous pressure change rate.

3. The apparatus (10) according to claim 1, wherein the control circuitry (11) is configured to estimate the amount of exudate by:
comparing a pressure change rate as output by the first pressure sensor (15a) after opening the electronically controllable valve (40) with a set pressure change rate value;
estimating the amount of exudate based on the comparison between the pressure change rate and the set pressure change rate value.

4. The apparatus (10) according to claim 1, further comprising:
a second pressure sensor (15b) configured to monitor a pressure level in the second fluid flow path;
wherein the control circuitry (11) is operatively connected to the second pressure sensor (15b) and configured to estimate the amount of exudate by:
comparing a first pressure level as output by the first pressure sensor (15a) after the source of negative pressure (14) has been active for a duration of time with a second pressure level as output by the second pressure sensor (15b) after the source of negative pressure (14) has been active for the duration of time,
estimating the amount of exudate based on the comparison between the first pressure level and the second pressure level.

5. The apparatus (10) according to claim 1, further comprising:
a second pressure sensor (15b) configured to monitor a pressure level in the second fluid flow path;
wherein the control circuitry (11) is operatively connected to the second pressure sensor (15b) and configured to estimate the amount of exudate by:
comparing a first pressure change rate as output by the first pressure sensor (15a) while the source of negative pressure (14) is active with a second pressure change rate as output by the second pressure sensor (15b) while the source of negative pressure is active,
estimating the amount of exudate based on a comparison between the first pressure change rate and the second pressure change rate.

6. The apparatus (10) according to claim 1, further comprising:
a second pressure sensor (15b) configured to monitor a pressure level in the second fluid flow path;
wherein the control circuitry (11) is operatively connected to the second pressure sensor (15b) and configured to estimate the amount of exudate by:
comparing a first pressure level as output by the first pressure sensor (15a) after opening the electronically controllable valve (40) with a second pressure level as output by the second pressure sensor (15b) after opening the electronically controllable valve (40),
estimating the amount of exudate based on the comparison between the first pressure level and the second pressure level.

7. The apparatus (10) according to claim 1, further comprising:
a second pressure sensor (15b) configured to monitor a pressure level in the second fluid flow path;
wherein the control circuitry (11) is operatively connected to the second pressure sensor (15b) and configured to estimate the amount of exudate by:
comparing a first pressure change rate as output by the first pressure sensor (15a) after opening the electronically controllable valve (40) with a second pressure change rate as output by the second pressure sensor (15b) after opening the electronically controllable valve (15b);
estimating the amount of exudate based on the comparison between the first pressure change rate and the second pressure change rate.

8. The apparatus (10) according to any one of claims 1-7, wherein the control circuitry (11) is configured to estimate the amount of exudate by:
counting a number of activations of the source of negative pressure (14) during at least a portion of an inter-flushing period,
comparing the number of counted activations of the source of negative pressure (14) with a set value,
estimating the amount of exudate based on the comparison between the number of counted activations and the set value.

9. A method (S100) for operating an apparatus for providing negative pressure to a wound site, wherein the apparatus comprises a source of negative pressure configured to provide negative pressure via a first fluid flow path from an inlet of the source of negative pressure to a wound site, a canister for receiving fluids from the wound site, and an electronically controllable valve configured to release negative pressure from the wound site via a second fluid flow path, the method comprising:
estimating (S101) an amount of exudate to be moved from the wound site via a portion of the first fluid flow path to the canister;
controlling (S111, S112) a duration of a flushing period and/or a duration of an inter-flushing period based on the estimated amount of exudate;
wherein the flushing period defines a time period during which the electronically controllable valve is opened and the source of negative pressure is activated for transporting fluid from the wound site to the canister, and the inter-flushing period defines a time between two consecutive flushing periods.

10. The method (S100) according to claim 9, wherein the estimating (S101) an amount of exudate to be moved from the wound site via a portion of the first fluid flow path to the canister comprises:
comparing (S102) a pressure change rate as detected in the first fluid flow path while the source of negative pressure is active with a previous pressure change rate as detected in the first fluid flow path while the source of negative pressure was active during a preceding time period,
estimating the amount of exudate based on the comparison between the pressure change rate and the previous pressure change rate.

11. The method (S100) according to claim 9, wherein the estimating (S101) an amount of exudate to be moved from the wound site via a portion of the first fluid flow path to the canister comprises:
comparing (S105) a pressure change rate as detected in the first fluid flow path after opening the electronically controllable valve with a set pressure change rate value;
estimating the amount of exudate based on the comparison between the pressure change rate and the set pressure change rate value.

12. The method (S100) according to any one of claims 9-11, wherein the estimating (S101) an amount of exudate to be moved from the wound site via a portion of the first fluid flow path to the canister comprises:
counting a number of activations of the source of negative pressure during at least a portion of an inter-flushing period,
comparing (S110) the number of counted activations of the source of negative pressure with a set value,
estimating the amount of exudate based on the comparison between the number of counted activations and the set value.

13. A computer program product comprising instructions which, when the program is executed by a computing device of an apparatus for providing negative pressure to a wound site, causes the apparatus to carry out the method (S100) according to any one of claims 9-12.

14. A wound treatment system (1) comprising:
an apparatus (10) according to any one of claims 1-8;
a wound cover (22) for creating a sealed space defined in part by the wound site; and
a tubing assembly defining the first fluid flow path and the second fluid flow path.
